# FASCICULE DE BREVET EUROPEEN

(11) **EP 0 911 062 B1**
(45) Date de publication et mention de la délivrance du brevet: **01.09.2004**
(21) Numéro de dépôt: 98402695.5
(22) Date de dépôt: 28.10.1998
(51) Int. Cl.: A61N 1/362

(54) **Stimulateur cardiaque multisites programmable**
Programmierbarer Herzschrittmacher zur Stimulation mehrerer Herzbereiche
Programmable multi-site cardiac stimulator

(30) Priorité: 28.10.1997 FR 9713493
(43) Date de publication de la demande: 28.04.1999
(73) Titulaire: ELA MEDICAL (Société anonyme), 92541 Montrouge (FR)
(72) Inventeur: Ripart, Alain, 91190 Gif sur Yvette (FR); Dal Molin, Renzo, 92320 Chatillon (FR)
(74) Mandataire: Dupuis-Latour, Dominique

(56) Documents cités:
- EP-A- 0 676 217
- WO-A-98/09680
- US-A- 4 549 548
- US-A- 4 928 688
- US-A- 5 403 356
- US-A- 5 439 482

## Description

La présente invention concerne les stimulateurs cardiaques (ou les circuits de stimulation d'un défibrillateur ou cardioverteur) dans leur utilisation pour le traitement de l'insuffisance cardiaque par stimulation.

En effet, outre le traitement des troubles du rythme cardiaque, il a été proposé de traiter par stimulation les troubles de contraction myocardique observés chez des patients en insuffisance cardiaque, ces troubles pouvant être spontanés ou induits par une stimulation traditionnelle. On pourra notamment se référer à l'étude de J. C. Daubert et coll., *Stimucoeur,* Tome 25, n°3, pp. 170-176 qui fait le point des travaux sur ce sujet.

Il a été notamment proposé de stimuler les cavités gauche et droite dans des modèles dits "multisites", c'est-à-dire des stimulateurs dans lesquels des électrodes sont placées en une pluralité de sites respectifs distincts, avec au moins un site ventriculaire. Il peut s'agir d'une double stimulation ventriculaire, d'une double stimulation ventriculaire avec stimulation atriale droite (modèles "triple chambre") ou même d'une double stimulation ventriculaire avec double stimulation atriale (modèles "quadruple chambre").

La stimulation des deux sites ventriculaires doit être simultanée, et l'on utilise généralement à cet effet un stimulateur de type classique comportant un connecteur bipolaire pour la stimulation ventriculaire, sur lequel on monte une sonde particulière comportant un adaptateur en forme de Y, avec d'un côté (hampe du Y) une liaison bipolaire jusqu'au connecteur du stimulateur, et de l'autre côté (chacune des branches du Y) deux sondes unipolaires correspondant aux deux conducteurs respectifs de la partie bipolaire et aboutissant chacune à l'un des deux sites ventriculaires, droit et gauche.

Une telle configuration permet de transformer la sortie bipolaire d'un stimulateur classique en deux sorties unipolaires simultanées.

Toutefois, l'utilisation d'un tel connecteur en Y n'est pas dépourvue d'inconvénients, que ce soit au moment de l'implantation puisqu'il faut mettre en place et loger ce connecteur, ou ultérieurement en termes de fiabilité, notamment du fait de la difficulté à maintenir une étanchéité parfaite à l'égard des fluides organiques environnant le connecteur.

De plus ce montage, une fois mis en place, produit systématiquement une double stimulation (des deux sites ventriculaires), sans qu'il soit possible ultérieurement de supprimer l'une des stimulations, par exemple suite à l'élévation de seuil sur l'une des sondes, sauf bien entendu dans le cadre d'une nouvelle intervention.

Il a également été proposé, par exemple par le US-A-4 928 688 de dédoubler les circuits de stimulation ventriculaire, ce qui implique bien entendu une multiplication correspondante du nombre de composants et de la surface qu'ils occupent sur le circuit.

L'un des buts de l'invention est de remédier à ces divers inconvénients, en permettant de réaliser une double stimulation simultanée de deux sites ventriculaires (et/ou de deux sites auriculaires) sans aucun connecteur intermédiaire en "Y", en évitant donc toutes les difficultés liées à cette connectique particulière, ni dédoublement des étages de stimulation.

Un autre but de l'invention est de proposer un dispositif qui autorise une commande de l'une ou l'autre des stimulations, c'est-à-dire qui permette par exemple une programmation à volonté entre une double stimulation ventriculaire, une stimulation du seul ventricule droit ou encore une stimulation du seul ventricule gauche, ce choix étant opéré de manière non invasive et au moyen d'un appareil connu tel qu'un programmateur associé au stimulateur.

Enfin, on verra que l'invention peut être mise en oeuvre à partir d'un stimulateur classique d'un modèle préexistant, les modifications n'étant apportées qu'à l'étage de sortie avec adjonction d'un circuit additionnel, donc sans nécessiter une nouvelle étape de conception du circuit principal du stimulateur ni du logiciel de commande de celui-ci.

Pour atteindre ces buts, l'invention propose un stimulateur de type multisite comprenant les caractéristiques énoncées par les revendications 1 (double stimulation ventriculaire) ou 2 (double stimulation atriale). Les sous-revendications visent des modes de réalisation avantageux.

On va maintenant décrire un exemple de mise en oeuvre de l'invention, en référence à la figure unique annexée qui est un schéma par blocs d'un stimulateur mettant en oeuvre les enseignements de l'invention.

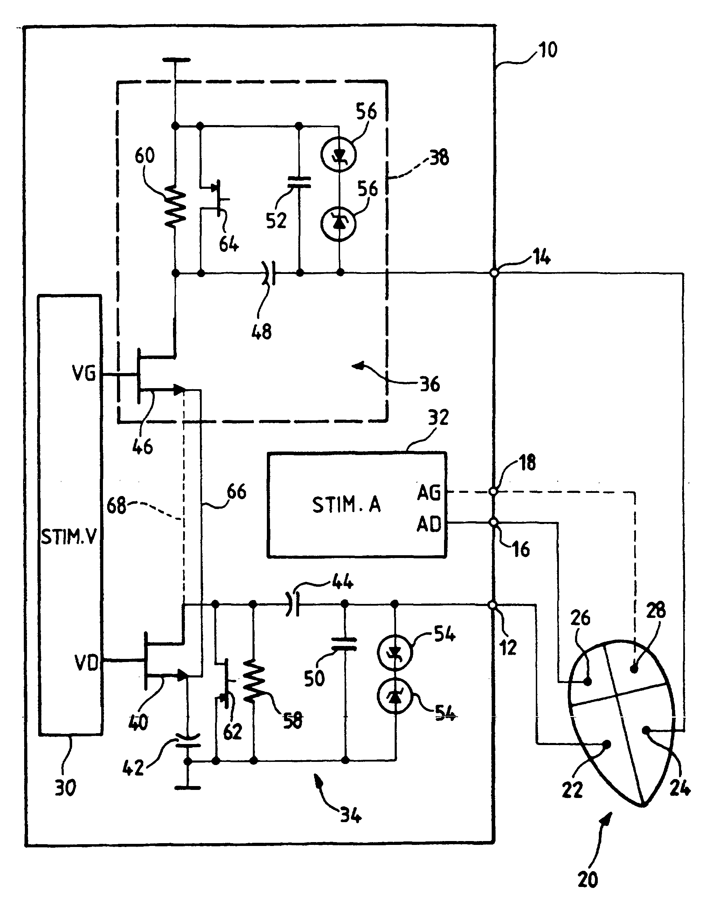

Sur la figure 1, la référence 10 désigne les circuits d'un stimulateur cardiaque, qui est dans cet exemple du type "quadruple chambre" avec quatre sorties monopolaires 12, 14, 16, 18 reliées par une ou plusieurs sondes appropriées au myocarde 20 en des sites respectifs 22 (ventricule droit), 24 (ventricule gauche), 26 (oreillette droite) et 28 (oreillette gauche).

Il est prévu un circuit de stimulation ventriculaire 30 avec deux sorties VD et VG pour la stimulation respective des ventricules droit et gauche, et un circuit de stimulation auriculaire 32 pourvu de deux sorties AD et AG pour la stimulation respective des oreillettes droite et gauche.

Dans une version simplifiée de l'invention, le stimulateur peut être seulement du type "triple chambre", c'est-à-dire dans lequel on ne stimule qu'une seule des oreillettes, l'oreillette droite. Dans une autre variante encore, la stimulation peut être limitée aux ventricules et il n'est alors pas prévu de circuit de stimulation auriculaire.

Le stimulateur comporte par ailleurs, de manière en elle-même connue, des circuits de détection des signaux cardiaques ventriculaires et auriculaires, qui ne seront pas décrits en détail dans la mesure où l'invention ne concerne que l'aspect stimulation, pour le traitement des troubles du rythme cardiaque.

Le circuit de stimulation ventriculaire 30 peut être un circuit d'un appareil conventionnel, non modifié, notamment un circuit de stimulation ventriculaire en mode bipolaire programmable, c'est-à-dire que le logiciel de commande du stimulateur permet au praticien de choisir un mode de fonctionnement monopolaire (signal présent sur un seul des deux pôles) ou bipolaire (signal présent sur les deux pôles). Les deux sorties de ce circuit correspondent alors, dans le cadre de l'invention, aux sorties désignées VD et VG sur la figure.

Il peut en être de même, le cas échéant, pour le circuit de stimulation auriculaire 32, qui opérera de la même manière que le circuit 30 et avec les mêmes composants annexes que l'on indiquera plus bas ; on ne décrira donc pas plus en détail ce circuit de stimulation auriculaire 32.

La sortie VD est reliée à un étage de sortie 34 qui pilote la sonde montée sur le connecteur 12, et la sortie VG est reliée à un circuit 36 qui pilote la sonde montée sur le connecteur 14.

Le circuit 34 peut être l'étage de sortie préexistant d'un stimulateur classique, qui n'aura été que très légèrement modifié pour la mise en oeuvre de l'invention. Le circuit 36, quant à lui, est constitué d'un petit nombre de composants additionnels, qui peuvent être montés sur un circuit souple 38 adjoint au circuit principal préexistant portant tous les composants du stimulateur.

Lorsque l'on souhaite stimuler le ventricule droit, un signal VD est délivré par le circuit 30, rendant passant le transistor 40, ce qui permet un transfert de charge d'un condensateur 42, monté entre la source du transistor 40 et la masse et chargé préalablement à une tension appropriée, vers un condensateur 44, monté quant à lui entre le drain du transistor 40 et la borne 12 à laquelle est reliée la sonde aboutissant au site 22 de stimulation du ventricule droit.

Lorsque l'on souhaite stimuler le ventricule gauche, un signal VG est délivré par le circuit 30, rendant passant le transistor 46, ce qui permet un transfert de charge du condensateur 42, chargé préalablement à une tension appropriée, vers un condensateur 48, monté quant à lui entre le drain du transistor 46 et la borne 14 à laquelle est reliée la sonde aboutissant au site 24 de stimulation du ventricule gauche.

Lorsque l'on souhaite stimuler à la fois ventricule gauche et ventricule droit, le circuit 30 délivre sur VD et VG des signaux qui rendent passants les deux transistors 40 et 46. Ceci a pour effet de transférer la charge du condensateur 42 précité à la fois vers le condensateur 44 et le condensateur 48 précités.

Afin d'obtenir un transfert de charge équilibré, la capacité de chacun des condensateurs 44 et 48 est de l'ordre de la moitié de celle du condensateur 42.

Divers circuits auxiliaires peuvent être prévus, comme :
- des condensateurs 50 et 52 de filtrage des interférences électromagnétiques, montés respectivement entre les bornes 12 et 14 et la masse ;
- des diodes Zener montées en opposition 54, 54 et 56, 56, également montées entre les bornes respectives 12 et 14 et la masse, pour écrêter les tensions dues aux perturbations susceptibles d'apparaître sur les sondes reliant ces bornes au myocarde ;
- des résistances 58 et 60 montées entre la masse et les condensateurs respectifs 44 et 48, pour ajuster le profil de décharge du condensateur par le choix d'une constante de temps RC appropriée ;
- des transistors 62 et 64 permettant de court-circuiter ces résistances 58 et 60, et donc de mettre fin à la décharge, après une durée prédéterminée, programmée par le stimulateur.

Grâce à la configuration que l'on vient de décrire, il est possible, au choix, soit de stimuler simultanément le ventricule gauche et le ventricule droit en mode unipolaire, lorsque le mode de stimulation "bipolaire" est programmé dans le circuit 30, soit de stimuler un seul ventricule, le droit ou bien le gauche, en mode unipolaire également, lorsque le mode de stimulation "unipolaire" est programmé dans le circuit 30.

Lorsque l'on veut stimuler simultanément ventricule droit et ventricule gauche, il se présente deux possibilités : soit les commandes VG et VD sont confondues, c'est-à-dire les mêmes, soit la commande VG englobe la commande VD, en d'autres termes la commande VG est plus large que la commande VD.

Il est intéressant de disposer du choix indiqué plus haut, non seulement au moment de l'implantation, mais également en cours de fonctionnement. En effet, si le dispositif est par exemple programmé en mode "bipolaire", c'est-à-dire s'il y a stimulation des deux ventricules gauche et droit et que l'on observe une élévation progressive du seuil sur la sonde gauche, il suffit de reprogrammer le stimulateur en mode "monopolaire" pour mettre hors service l'un des circuits de stimulation et stimuler un seul des ventricules.

En variante, si l'on remplace la liaison 66 entre les sources des transistors 40 et 46 par une liaison 68 entre la source du transistor 46 et le drain du transistor 40, on dispose d'une configuration permettant, au choix, ou bien de stimuler simultanément le ventricule gauche et le ventricule droit en mode unipolaire, lorsque le mode de stimulation "bipolaire" est programmé dans le circuit 30, ou bien de stimuler le seul ventricule droit, en mode unipolaire également, lorsque le mode de stimulation "unipolaire" est programmé dans le circuit 30.

Dans encore une autre variante, afin de rendre le circuit de stimulation gauche indépendant du circuit de stimulation droit, les conducteurs 66 et 68 sont supprimés et un condensateur de même capacité que le condensateur 42 est monté entre la source du transistor 46 et la masse.

Bien entendu, comme on l'a indiqué plus haut, tout ce qui vient d'être exposé à propos de la stimulation ventriculaire droite et gauche est directement transposable à la stimulation auriculaire droite et gauche, pour la mise en oeuvre d'un stimulateur triple ou quadruple chambre.

## Revendications

1. Un stimulateur cardiaque multisite, comprenant :
- une pluralité de sorties, aptes à être reliées à des électrodes de stimulation placées sur une pluralité de sites distincts d'un myocarde,
- un circuit de recueil de signaux cardiaques, apte à détecter un potentiel de dépolarisation sur au moins l'un desdits sites,
- un circuit de stimulation (30), apte à commander si nécessaire la délivrance d'une impulsion de stimulation sur deux sites ventriculaires desdits sites,
stimulateur **caractérisé en ce que** lesdites sorties comprennent:
- une première sortie (12) apte à être reliée à une électrode de stimulation placée sur un site ventriculaire droit (22) du myocarde,
- une deuxième sortie (14), distincte de la première, apte à être reliée à une électrode de stimulation placée sur un site ventriculaire gauche (24) du myocarde,
- éventuellement une troisième sortie (16, 18), apte à être reliée à une électrode de stimulation placée sur un site atrial (26, 28) du myocarde,
et **en ce qu'**il est en outre prévu des moyens sélecteurs (34, 36), disposés à l'intérieur du stimulateur, configurables pour relier sélectivement au circuit de stimulation (30), qui leur est commun, soit la première sortie (12), soit la deuxième sortie (14), soit à la fois la première et la deuxième sortie (12, 14).

2. Un stimulateur cardiaque multisite, comprenant :
- une pluralité de sorties, aptes à être reliées à des électrodes de stimulation placées sur une pluralité de sites distincts d'un myocarde,
- un circuit de recueil de signaux cardiaques, apte à détecter un potentiel de dépolarisation sur au moins l'un desdits sites,
- un circuit de stimulation (32), apte à commander si nécessaire la délivrance d'une impulsion de stimulation sur au moins deux sites atriaux desdits sites,
stimulateur **caractérisé en ce que** lesdites sorties comprennent:
- une première sortie (16) apte à être reliée à une électrode de stimulation placée sur un site atrial droit (26) du myocarde,
- une deuxième sortie (18), distincte de la première, apte à être reliée à une électrode de stimulation placée sur un site atrial gauche (28) du myocarde,
- une troisième sortie (12, 14), apte à être reliée à une électrode de stimulation placée sur un site ventriculaire (12, 14) du myocarde,
et **en ce qu'**il est en outre prévu des moyens sélecteurs, disposés à l'intérieur du stimulateur, configurables pour relier sélectivement au circuit de stimulation (32), qui leur est commun, soit la première sortie (16), soit la deuxième sortie (18), soit à la fois la première et la deuxième sortie (16, 18).

3. Le stimulateur de la revendication 1 ou 2, dans lequel, lorsque les moyens sélecteurs relient les deux sorties, droite et gauche, au circuit de stimulation, ils appliquent simultanément à ces deux électrodes l'impulsion de stimulation.

4. Le stimulateur de la revendication 1 ou 2, dans lequel la configuration des moyens sélecteurs est définie par un programmateur couplé temporairement au stimulateur par télémétrie.

5. Le stimulateur de la revendication 1 ou 2, dans lequel les composants additionnels constituant lesdits moyens sélecteurs sont montés sur un circuit souple rapporté (36) relié au circuit principal du stimulateur, ce circuit principal portant notamment les composants du circuit de stimulation.

6. Le stimulateur de la revendication 1 ou 2, dans lequel le circuit de stimulation est un circuit programmable en mode de stimulation monopolaire ou bipolaire, et les moyens sélecteurs appliquent l'impulsion de stimulation à une seule des sorties lorsque la programmation du circuit est monopolaire, et aux deux sorties lorsque la programmation du circuit est bipolaire.

## Patentansprüche

1. Herzschrittmacher zur Stimulation mehrerer Herzbereiche, umfassend:
- eine Vielzahl von Ausgängen, geeignet, um mit Stimulationselektroden verbunden zu werden, die an einer Vielzahl von unterschiedlichen Stellen eines Herzmuskels platziert sind,
- einen Schaltkreis zum Empfangen von Herzsignalen, geeignet, um ein Depolarisations-Potential an mindestens einer der Stellen zu detektieren,
- einen Stimulationsschaltkreis (30), geeignet, wenn nötig die Abgabe eines Stimulationsimpulses an zwei ventrikulären Stellen der Stellen zu steuern,
wobei der Schrittmacher **dadurch gekennzeichnet ist, dass** die Ausgänge umfassen:
- einen ersten Ausgang (12), geeignet, um mit einer Stimulationselektrode verbunden zu werden, die an einer rechten ventrikulären Stelle (22) des Herzmuskels platziert ist,
- einen zweiten Ausgang (14), unterschiedlich von dem ersten, geeignet, um mit einer Stimulationselektrode verbunden zu werden, welche an einer linken ventrikulären Stelle (24) des Herzmuskels platziert ist,
- eventuell einen dritten Ausgang (16, 18), geeignet, um mit einer Stimulationselektrode verbunden zu werden, die an einer atrialen Stelle (26, 28) des Herzmuskels platziert ist,
und dadurch, dass ferner Auswahlmittel (34, 36) vorgesehen sind, angebracht im Inneren des Stimulators, konfigurierbar, um selektiv mit dem Stimulationsschaltkreis (30) zu verbinden, der ihnen gemeinsam ist, entweder den ersten Ausgang (12), den zweiten Ausgang (14) oder gleichzeitig den ersten und zweiten Ausgang (12, 14).

2. Herzschrittmacher zur Stimulation mehrerer Herzbereiche, umfassend:
- eine Vielzahl von Ausgängen, geeignet, um mit Stimulationselektroden verbunden zu werden, die an einer Vielzahl von unterschiedlichen Stellen eines Herzmuskels platziert sind,
- einen Schaltkreis zum Empfangen von Herzsignalen, geeignet, um ein Depolarisations-Potential an mindestens einer der Stellen zu detektieren,
- einen Stimulationsschaltkreis (32), geeignet, wenn nötig die Abgabe eines Stimulationsimpulses an mindestens zwei atrialen Stellen der Stellen zu steuern,
wobei der Schrittmacher **dadurch gekennzeichnet ist, dass** die Ausgänge umfassen:
- einen ersten Ausgang (16), geeignet, um mit einer Stimulationselektrode verbunden zu werden, die an einer rechten atrialen Stelle (26) des Herzmuskels platziert ist,
- einen zweiten Ausgang (18), unterschiedlich von dem ersten, geeignet, um mit einer Stimulationselektrode verbunden zu werden, welche an einer linken atrialen Stelle (28) des Herzmuskels platziert ist,
- einen dritten Ausgang (12, 14), geeignet, um mit einer Stimulationselektrode verbunden zu werden, die an einer ventrikulären Stelle (12, 14) des Herzmuskels platziert ist,
und dadurch, dass ferner Auswahlmittel vorgesehen sind, die im Inneren des Schrittmachers angebracht sind, konfigurierbar, um selektiv mit dem Stimulationsschaltkreis (32), der ihnen gemeinsam ist, entweder den ersten Ausgang (16), oder den zweiten Ausgang (18), oder gleichzeitig den ersten und zweiten Ausgang (16, 18) zu verbinden.

3. Schrittmacher nach Anspruch 1 oder 2, in welchem, wenn die Auswahlmittel die zwei Ausgänge, rechter und linker, mit dem Stimulationsschaltkreis verbinden, sie gleichzeitig den Stimulationsimpuls an diese zwei Elektroden anlegen.

4. Schrittmacher nach Anspruch 1 oder 2, in welchem die Konfiguration der Auswahlmittel durch ein Programmiergerät definiert wird, welches zeitweilig mit dem Stimulator über Telemetrie gekoppelt ist.

5. Schrittmacher nach Anspruch 1 oder 2, in welchem die zusätzlichen Bestandteile, welche die Auswahlmittel bilden, auf einem biegsamen Zusatzschaltkreis (36) montiert sind, welcher mit dem Hauptschaltkreis des Schrittmachers verbunden ist, wobei der Hauptschaltkreis insbesondere die Bestandteile des Stimulationsschaltkreises trägt.

6. Schrittmacher nach Anspruch 1 oder 2, in welchem der Stimulationsschaltkreis ein im monopolaren oder bipolaren Stimulationsmodus programmierbarer Schaltkreis ist, und die Auswahlmittel den Stimulationsimpuls an einen einzigen Eingang anlegen, wenn die Programmierung des Schaltkreises monopolar ist und an zwei Ausgänge, wenn die Programmierung des Schaltkreises bipolar ist.

## Claims

1. A multisite cardiac pacemaker, comprising:
- a plurality of outputs, adapted to be connected to stimulation electrodes located on a plurality of distinct sites of a myocardium,
- a cardiac signal collection circuit, adapted to detect a depolarization potential on at least one of said sites,
- a stimulation circuit (30) adapted to control, when necessary, the application of a stimulation pulse on two ventricular sites among said sites,
said pacemaker being **characterized in that** said outputs include :
- a first output (12) adapted to be connected to a stimulation electrode located on a right ventricular site (22) of the myocardium,
- a second output (14), different from the first one, adapted to be connected to a stimulation electrode located on a left ventricular site (24) of the myocardium,
and it that there is further provided selection means (34, 36), located inside the pacemaker, that can be configured to selectively connect to the stimulation circuit (30), which is common to them, either the first output (12), or the second output (14), or both the first and the second output (12, 14).

2. A multisite cardiac pacemaker, comprising:
- a plurality of outputs, adapted to be connected to stimulation electrodes located on a plurality of distinct sites of a myocardium,
- a cardiac signal collection circuit, adapted to detect a depolarization potential on at least one of said sites,
- a stimulation circuit (32) adapted to control, when necessary, the application of a stimulation pulse on two atrial sites among said sites,
said pacemaker being **characterized in that** said outputs include :
- a first output (16) adapted to be connected to a stimulation electrode located on a right atrial site (26) of the myocardium,
- a second output (18), different from the first one, adapted to be connected to a stimulation electrode located on a left atrial site (28) of the myocardium,
and it that there is further provided selection means, located inside the pacemaker, that can be configured to selectively connect to the stimulation circuit (32), which is common to them, either the first output (16), or the second output (18), or both the first and the second output (16, 18).

3. The pacemaker of claim 1 or 2, wherein, when the selection means connects the two outputs, left and right, to the stimulation circuit, it applies simultaneously the stimulation pulse to these two electrodes.

4. The pacemaker of claim 1 or 2, wherein the arrangement of the selection means is defined by a programmer temporarily coupled to the pacemaker by telemetry.

5. The pacemaker of claim 1 or 2, wherein the additional devices making up said selection means are mounted on a add-on flexible circuit board (36) connected to the main circuit board of the pacemaker, said main circuit board in particular supporting the devices of the stimulation circuit.

6. The pacemaker of claim 1 or 2, wherein the stimulation circuit is a circuit that can be programmed in unipolar or bipolar stimulation mode, and the selection means applies the stimulation pulse only to one of the outputs when the programming of the circuit is a unipolar one, and to both outputs when the programming of the circuit is a bipolar one.
